# EUROPÄISCHE PATENTSCHRIFT

(11) **EP 1 437 990 B1**
(45) Veröffentlichungstag und Bekanntmachung des Hinweises auf die Patenterteilung: **12.12.2007**
(21) Anmeldenummer: 02801860.4
(22) Anmeldetag: 28.08.2002
(51) Int. Cl.: A61F 2/44

(54) **IMPLANTAT**
IMPLANT
IMPLANT

(30) Priorität: 24.10.2001 DE 10152567
(43) Veröffentlichungstag der Anmeldung: 21.07.2004
(73) Patentinhaber: Tutogen Medical GmbH, 91077 Neunkirchen am Brand (DE)
(72) Erfinder: KRÜGER, Manfred, 61389 Arnoldshain (DE)
(74) Vertreter: Manitz, Finsterwald & Partner GbR
(86) Internationale Anmeldenummer: PCT/EP2002/009602
(87) Internationale Veröffentlichungsnummer: WO 2003/034956

(56) Entgegenhaltungen:
- WO-A-01/64141
- WO-A-01/70137
- US-A- 6 025 538
- US-B1- 6 174 311

## Beschreibung

Die vorliegende Erfindung betrifft ein Wirbelsäulenimplantat zur interkorporellen Fusion an der Wirbelsäule.

Durch die Degeneration der Bandscheibe, insbesondere des Bandscheibenkerns, kommt es zu einem Höhenverlust im betroffenen Bandscheibenfach, der mit einer Lockerung des Bandscheibenringes und der Bänder verbunden ist. Hierdurch wird die Wirbelsäule an dieser Stelle instabil.
Die Folge ist eine horizontale Verschiebbarkeit der Wirbelkörper gegeneinander, die zu Beeinträchtigungen der Nervenwurzeln in diesem Bereich und/oder des Rückenmarks mit daraus resultierenden Schmerzen führt.

Das Prinzip zur Behandlung dieser Symptome besteht in der operativen Ausräumung des Bandscheibenkerns und dem Einsetzen von Stützkörpern, um die normale Höhe des Bandscheibenfaches wiederherzustellen.

Die Anforderungen an Wirbelsäulenimplantate und insbesondere an Implantate zur interkorporellen Fusion der Lendenwirbelsäule sind vielfältig. So soll zum einen eine möglichst große Kontaktfläche mit den zu fusionierenden Wirbelkörpern vorgesehen werden. Dies bedeutet, dass bei Verwendung herkömmlicher Implantate eine Vielzahl von Implantaten vorrätig gehalten werden muss, da präoperativ die erforderlichen Maße nicht genau zu ermitteln sind und da die Länge und die Höhe der Wirbelkörper variiert. Untersuchungen haben nämlich gezeigt, dass die Länge der Wirbelkörper zwischen 22 und 34 mm und ihre Breite zwischen 31 und 49 mm schwanken kann (vgl. Ebraheim et al, Anatomic Considerations for Anterior Instrumentation of the Lumbar Spine, ORTHOPEDICS, October 1999, Volume 22 No. 10).

Eine weitere Anforderung an Wirbelsäulenimplantate und insbesondere Implantate für die Lendenwirbelsäule ist eine ausreichende und ausreichend vorhersagbare Druckfestigkeit. Darüber hinaus soll das Wirbelsäulenimplantat möglichst die gleiche Elastizität wie der Knochen haben, was bei herkömmlichen Metallimplantaten nicht der Fall ist.

Aus der DE 199 52 939 A1 ist ein Wirbelsäulenimplantat zur interkorporellen Fusion an der Wirbelsäule bekannt, das aus einem Körper aus Knochenmaterial besteht, der in Richtung seiner Längserstreckung gekrümmt ausgebildet ist.

Die WO 01/70137 A2 beschreibt ein Implantat, welches aus zwei oder mehr Knochenfragmenten besteht, welche zusammengefügt werden, um eine einzige Einheit zu bilden. Die einzelnen Knochenfragmente werden dabei in ihrer Gestalt einzeln aufeinander abgestimmt, um zusammengefügt zu werden.

Es ist die Aufgabe der Erfindung, ein derartiges Wirbelsäulenimplantat dahingehend weiterzubilden, dass die eingangs genannten Anforderungen erfüllt werden, und dass insbesondere die Anzahl der vorrätig zu haltenden Implantatkörper reduziert werden.

Die Lösung dieser Aufgabe erfolgt durch ein modulares System zur interkorporellen Fusion an der Wirbelsäule mit den Merkmalen des Anspruchs 1. Die Lösung der Aufgabe erfolgt insbesondere dadurch, dass das modulare System aus zumindest einem im weiteren Verlauf noch genauer zu beschreibenden Basiskörper und mehreren Verlängerungsteilen besteht, die jeweils mit einem geeigneten Kopplungsmittel versehen sind, wobei die Verlängerungsteile unterschiedliche Längen und/oder Krümmungen aufweisen.

Durch ein solches modulares System lassen sich Wirbelsäulenimplantate der unterschiedlichsten Abmessung und Gestaltung zusammenstellen, ohne dass eine entsprechend große Anzahl an Implantaten bevorratet werden muss, da die jeweils erforderliche Form und Größe durch Zusammenstellen von entsprechendem Basiskörper und Verlängerungsteil(en) angepasst werden kann.

An zumindest einem Ende des Basiskörpers ist erfindungsgemäß ein Kopplungsmittel vorgesehen, das einstückig mit dem Körper ausgebildet ist, und das zur Ankopplung des mit einem komplementären Kopplungsmittel versehenen Verlängerungsteils dient, um das Wirbelsäulenimplantat in Richtung seiner Längserstreckung zu verlängern.

Erfindungsgemäß ist das Wirbelsäurenimplantat als Basisköper ausgebildet, der ein Kopplungsmittel aufweist, um ein ebenfalls aus Knochenmaterial bestehendes Verlängerungsteil anzukoppeln. Durch Vorsehen von Verlängerungsteilen unterschiedlicher Länge und/oder Krümmung kann mit Hilfe des erfindungsgemäßen Basiskörpers eine Vielzahl von Implantatgestaltungen erreicht werden, wobei dennoch die Anzahl der vorrätig zu haltenden Implantatkörper gering gehalten werden kann.

Vorteilhafte Ausführungsformen der Erfindung sind in der Beschreibung, der Zeichnung sowie den Unteransprüchen beschrieben.

Nach einer vorteilhaften Ausführungsform bestehen der Körper und insbesondere auch das Verlängerungsteil aus kortikalem Knochenmaterial. Dies besitzt gegenüber den üblicherweise verwendeten Beckenkammkeilen eine einheitliche Druckfestigkeit pro Querschnittsflächeneinheit.

Nach einer weiteren vorteilhaften Ausführungsform kann das Kopplungsmittel zur formschlüssigen Verbindung mit dem komplementären Kopplungsmittel ausgebildet sein. Hierdurch ist es möglich, sowohl den Körper wie auch das Verlängerungsteil durch einfaches Ineinanderstecken der Kopplungsmittel miteinander zu verbinden, wobei dennoch eine sichere und feste Verbindung gewährleistet ist, die sich nach Implantation nicht von selbst lösen kann.

Nach einer weiteren vorteilhaften Ausführungsform sind an zumindest einem Ende des Körpers ein erstes und ein zweites Kopplungsmittel vorgesehen, wobei insbesondere ein Kopplungsmittel als Vorsprung und ein Kopplungsmittel als Aussparung ausgebildet ist. Bei dieser Ausführungsform befinden sich an einem Ende des Körpers sowohl ein Vorsprung wie auch eine Aussparung, wobei an dem zugehörigen Ende des Verlängerungsteils ein komplementärer Vorsprung und eine komplementäre Aussparung so vorgesehen sein können, dass der Körper und das Verlängerungsteil durch einfaches Ineinandersetzen formschlüssig und fest miteinander verbunden sind.

Bevorzugt sind an beiden Enden des Körpers jeweils ein erstes und ein zweites Kopplungsmittel vorgesehen, wobei ein Kopplungsmittel als Vorsprung und ein Kopplungsmittel als Aussparung ausgebildet ist. Hierbei können insbesondere der Vorsprung und die Aussparung an einem Ende des Körpers in unterschiedlichen Längsebenen des Körpers angeordnet sein. Bei dieser Ausführungsform ist einerseits eine universelle Verwendung des Körpers möglich, da dieser an seinen beiden Enden mit Verlängerungsteilen versehen werden kann. Andererseits ist eine besonders stabile Verbindung geschaffen, da Vorsprung und Aussparung in unterschiedlichen Ebenen des Körpers angeordnet sind.

Ein weiterer erfindungsgemäßer Gedanke besteht in der Kombination eines Wirbelsäulenimplantats mit einem oben beschriebenen Basiskörper und einem Verlängerungsteil aus Knochenmaterial, das mit zumindest einem weiteren komplementären Kopplungsmittel versehen ist. Hierbei kann bei miteinander verbundenem Körper und Verlängerungsteil die Krümmung des Körpers stetig in die Krümmung des Verlängerungsteils übergehen. Je nach Bedarf kann die Krümmung kontinuierlich oder aber mit wechselndem Vorzeichen verlaufen, das heißt es können durch Verwendung unterschiedlicher Verlängerungsteile Kreissegmente aber auch S-förmige Implantate geschaffen werden.

Bevorzugt weist der Körper und/ oder das Verlängerungsteil ein abgerundetes Ende auf. Alternativ kann der Körper an einem oder an beiden Enden mit einem Vorsprung versehen sein. Wird der Basiskörper ohne Verlängerungsteil verwendet, ist es möglich, den Vorsprung entweder zu belassen oder aber ihn durch Sägen oder auf andere Weise zu entfernen.

Sowohl der Körper wie auch das Verlängerungsteil können im Querschnitt entweder planparallel oder aber keilförmig oder trapezoid ausgeformt sein. Bei keilförmiger oder trapezoider Ausformung kann die Abwinkelung bei einer Ringsegmentform entweder radial oder entlang der längsten Sekante angelegt sein. Bei einer S-förmigen Verlängerung wird bevorzugt die Keil- bzw. Trapezform entlang der Längskante des S angelegt, wobei der höchste Punkt am abgerundeten Ende des Basisgliedes liegt. Bevorzugt setzt sich die Keil- bzw. Trapezform über die Verlängerungsstücke hinweg fort.

Bei einer ringförmigen Verlängerung kann die Abschrägung in radialer Richtung angelegt werden, wobei der höchste Punkt in der Mitte des äußeren Umfangs des Ringsegments liegt. In diesem Fall kann der Körper mit jeweils einem Vorsprung an seinen Enden versehen werden, wobei die beidseitig anzubringenden Verlängerungsstücke der Verjüngung entsprechend folgen.

Nach einer weiteren bevorzugten Ausführungsform weist das erste und das zweite Kopplungsmittel im Wesentlichen die gleiche Höhe auf, die vorzugsweise die halbe Höhe des Körpers beträgt. Hierdurch wird eine besonders stabile Verbindung zwischen Körper und Verlängerungsteil geschaffen.

Der Außenradius des in seiner Längserstreckung gekrümmten Körpers kann mindestens 12 mm betragen, wobei seine Höhe etwa 6 bis 18 mm betragen kann.

Sowohl der Basiskörper wie auch das Verlängerungsteil können an ihrer Unter- und/oder Oberseite glatt oder angeraut sein, wobei die Anrauung riffelartig oder noppenartig sein kann. Die Riffel oder Noppen können entweder stumpf oder auch scharfkantig bzw. spitz ausgebildet sein.

Nach einer weiteren vorteilhaften Ausführungsform können sowohl Basiskörper wie auch Verlängerungsteil mit vertikalen Durchbrüchen versehen sein, die das Einwachsen von Kallusknochen begünstigen oder mit autologem, allogenem oder xenogenem Knochenersatzmaterial oder wachsturnsförderndem Material gefüllt werden können. Die Form dieser Durchbrüche kann grundsätzlich beliebig, vorzugsweise jedoch kreisrund im Sinne einer Bohrung sein.

Nachfolgend wird die vorliegende Erfindung rein beispielhaft anhand vorteilhafter Ausführungsformen und unter Bezugnahme auf die beigefügten Zeichnungen beschrieben. Es zeigen:
- Fig. 1: eine perspektivische Ansicht einer Ausführungsform eines Basiskörpers;
- Fig. 2: eine Draufsicht auf einen weiteren Basiskörper, der mit einem Verlängerungsteil verbunden ist;
- Fig. 3: eine Draufsicht sowie eine Seitenansicht eines Basiskörpers mit verbundenem Verlängerungsteil mit keilförmigem Querschnitt;
- Fig. 4: eine vergrößerte Seitenansicht der Kopplungsmittel von Basiskörper und Verlängerungsteil; und
- Fig. 5 bis 8: verschiedene Anwendungsbeispiele zum Einsetzen eines erfindungsgemäßen Wirbelsäulenimplantates in ein Bandscheibenfach.

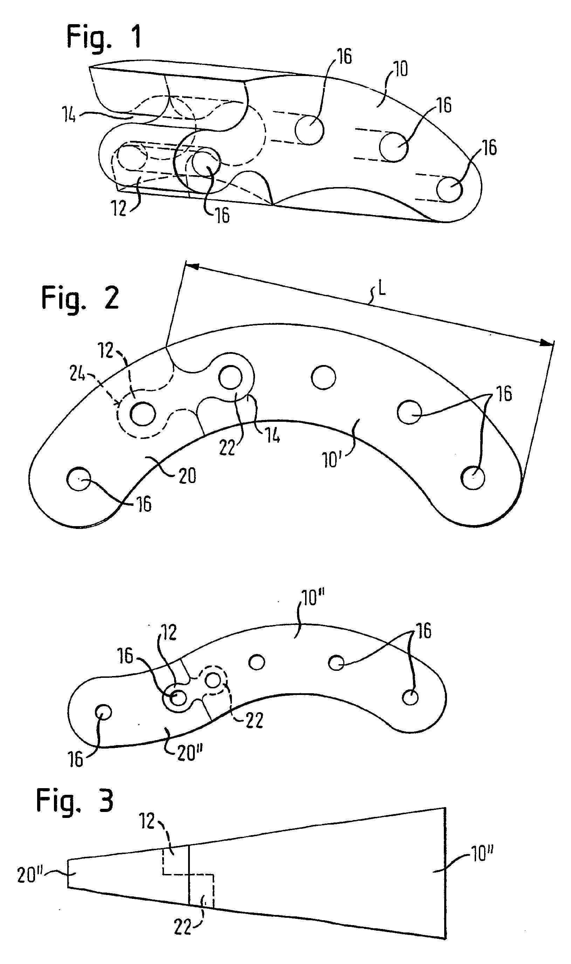

Fig. 1 zeigt eine perspektivische, schematische Ansicht eines Wirbelsäulenimplantates gemäß der Erfindung zur interkorporellen Fusion an der Wirbelsäule, das aus einem Körper bzw. Basiskörper 10 aus Knochenmaterial besteht, der grundsätzlich länglich ausgebildet ist und in Richtung seiner Längserstreckung gekrümmt ist. Der Basiskörper ist aus einem ausreichend dicken Knochen menschlichen oder tierischen Ursprungs als Ringsegment herausgeschnitten, wobei die Breite des Ringsegments mindestens 4 mm und der Außenradius des Ringsegments mindestens 12 mm betragen sollten. Die Höhe des Segments bzw. des Basiskörpers kann zwischen 6 und 18 mm liegen.

Wie Fig. 1 zeigt, sind an dem in Fig. 1 linken Ende des Basiskörpers 10 zwei Kopplungsmittel in Form eines Vorsprungs 12 und einer Aussparung 14 vorgesehen. Sowohl die Höhe des Vorsprungs 12 wie auch die Höhe der Aussparung 14 betragen die halbe Höhe des Basiskörpers 10. Der Vorsprung 12 ist zapfenartig ausgebildet und einstückig mit dem Basiskörper 10 verbunden. Die Form des Vorsprungs 12 kann grundsätzlich variieren, beispielsweise schwalbenschwanzförmig oder oval sein. Bevorzugt ist jedoch der Zapfen in seinem Querschnitt kreisrund ausgebildet, wobei die Rundung des Halses vorzugsweise den gleichen Radius aufweist, wie der Vorsprung selbst. Die Aussparung 14 ist komplementär zu dem Vorsprung ausgebildet, das heißt diese ist ebenfalls kreisrund und besitzt einen Hals, der den gleichen Radius wie die Rundung der Aussparung aufweist.

Das in Fig. 1 rechte Ende des Basiskörpers 10 ist abgerundet, vorzugsweise in Form eines Halbkreises. Alternativ können jedoch an dem Basiskörper an beiden Enden Kopplungsmittel, das heißt Vorsprünge und Aussparungen vorgesehen sein.

Ferner ist der Basiskörper 10 mit vertikal durchgehenden Bohrungen 16 versehen, die über die Längserstreckung des Basiskörpers 10 verteilt sind. Wie Fig. 1 zeigt, sind in dem Basiskörper 10 insgesamt vier Bohrungen 16 vorgesehen, wobei sich eine in der Mitte des Vorsprungs 12 befindet.

Fig. 2 zeigt eine Draufsicht auf eine weitere Ausführungsform eines Basiskörpers 10'. Dieser Basiskörper 10' ist im wesentlichen genauso wie der Basiskörper 10 ausgebildet, wobei jedoch die Lage von Vorsprung 12 und Aussparung 14 vertauscht ist, das heißt der Vorsprung 12 ist an der Unterseite und die Aussparung 14 ist an der Oberseite des Basiskörpers 10' vorgesehen.

Ferner ist der Basiskörper 10' mit einem Verlängerungsteil 20 verbunden, das mit komplementären Kopplungsmitteln, das heißt ebenfalls mit einem Vorsprung 22 und mit einer Aussparung 24 versehen ist, wobei Vorsprung 22 und Aussparung 24 so angeordnet sind, dass Basiskörper 10' und das Verlängerungsteil 20 formschlüssig ineinander gesteckt werden können.

Wie Fig. 2 zeigt, ist insgesamt ein Wirbelsäulenimplantat geschaffen, das ringsegmentförmig ausgebildet ist und das an seinen Enden kreisförmig abgerundet ist. Ferner befinden sich in der Mitte des kombinierten Wirbelsäulenimplantates insgesamt sechs durchgehende Bohrungen, die über die Längserstreckung des Wirbelsäulenimplantates gleichmäßig beabstandet sind. Die Längserstreckung L des Basiskörpers 10' beträgt zwischen den in gerader Linie am weitesten voneinander entfernten Punkten des Vollkörpers etwa 18 mm. Bei dem Verlängerungsteil 20 beträgt die Distanz zwischen den am weitesten voneinander entfernten Punkten des Vollkörpers etwa 4 mm bis etwa 12 mm. Bevorzugt werden mehrere Verlängerungsteile vorgesehen, wobei diese Distanz zwischen den einzelnen Verlängerungsteilen in 4 mm Schritten variiert.

Die Verlängerungsteile 20 werden grundsätzlich mit einem abgerundeten Ende ausgeführt. Das Ende mit Vorsprung und Aussparung wird bevorzugt in zwei Varianten ausgeführt, bei denen die Position von Vorsprung und Aussparungen vertauscht ist. Hierdurch ist es möglich, den Basiskörper 10 mit einem Verlängerungsteil wahlweise in Form eines Ringsegments oder in S-Form zu verlängern.

Fig. 3 zeigt eine Draufsicht auf eine solche Ausführungsform bei der ein Basiskörper mit einem Verlängerungsteil zu einer S-Form zusammengesteckt ist. Wie die Seitenansicht von Fig. 3 zeigt, ist bei dieser Ausführungsform sowohl der Basiskörper 10" wie auch das Verlängerungsteil 20" im Querschnitt keilförmig ausgebildet, wobei die Außenkontur des zusammengesetzten Wirbelsäulenimplantates stetig verläuft. Im übrigen sind gleiche Elemente mit gleichen Bezugszeichen versehen.

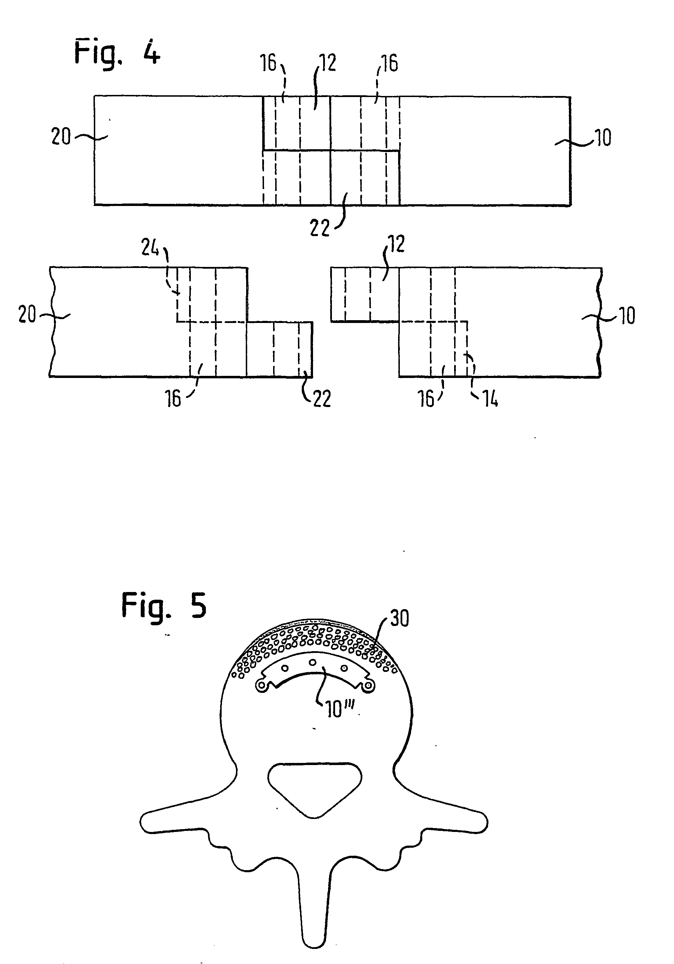

Fig. 4 zeigt eine Seitenansicht eines Basiskörpers 10 und eines Verlängerungsteils 20 im ineinander gesteckten und im auseinander gesteckten Zustand. Wie zu erkennen ist, bilden die Vorsprünge 12 und 22 des Basiskörpers 10 bzw. des Verlängerungsteils 20 jeweils eine Stufe, die der halben Höhe des Basiskörpers bzw. des Verlängerungsteils 20 im Bereich der Verbindung entspricht.

Die Fig. 5 bis 8 zeigen verschiedene Anwendungsbeispiele des oben beschriebenen Wirbelsäulenimplantates.

Fig. 5 zeigt eine Draufsicht auf einen Wirbelkörper, wobei die transforaminale lumbare interkorporelle Fusion (TLIF) dargestellt ist. Bei dieser Implantationstechnik wird ein Zwischenwirbelgelenk entfernt und dadurch das Foramen der Nervenwurzel freigelegt. Das ausgeräumte Bandscheibenfach wird im vorderen Drittel mit Knochenchips 30 gefüllt. Entlang dieser Knochenchips gleitet das Implantat in Form eines Basiskörpers 10'" in seine Position.

Der Basiskörper 10"' ist grundsätzlich wie oben beschrieben aufgebaut. Allerdings ist bei dieser Ausführungsform an beiden Enden des Basiskörpers 10'" eine Kopplungsmöglichkeit vorgesehen, das heißt an beiden Enden sind jeweils ein Vorsprung und eine Aussparung angeordnet. Falls es erforderlich ist, können die Vorsprünge an beiden Enden des Basiskörpers 10'" vor der Implantation entfernt werden.

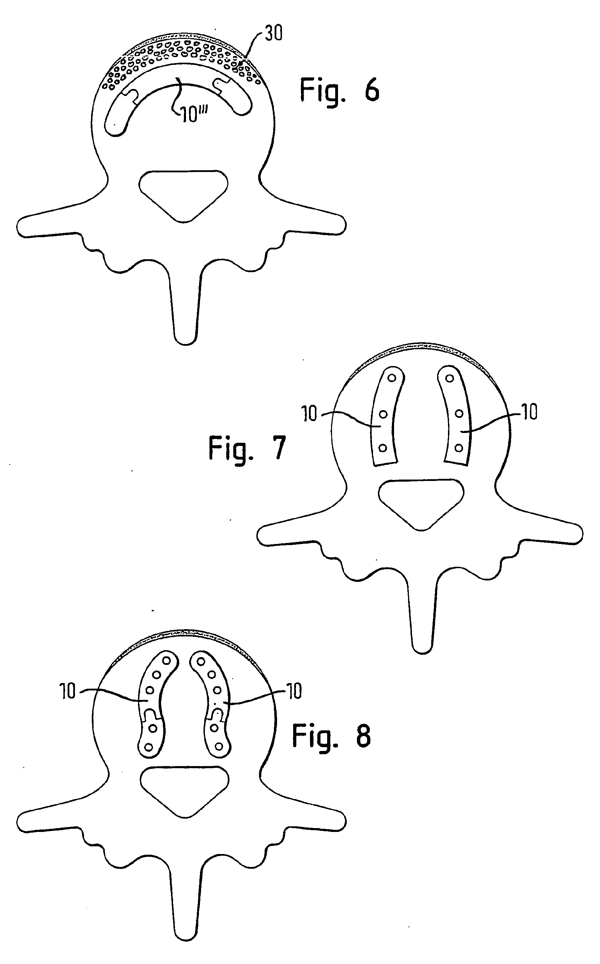

Fig. 6 zeigt eine zu Fig. 5 ähnliche Ansicht einer TLIF, wobei jedoch an beiden Enden des Basiskörpers 10'" ein Verlängerungsteil vorgesehen ist, das jeweils an seinem äußeren Ende abgerundet ist.

Fig. 7 zeigt ein Anwendungsbeispiel der posterioren lumbaren interkorporellen Fusion (PLIF), bei der zwei gleichartig ausgebildete Implantate jeweils links und rechts am Rückenmark vorbei in das Bandscheibenfach eingeschoben werden. In dem dargestellten Beispiel handelt es sich um zwei Basiskörper 10, bei denen jeweils die Vorsprünge 12 entfernt sind.

Schließlich ist in Fig. 8 ein weiteres Anwendungsbeispiel einer PLIF gezeigt, bei der die Basiskörper jeweils mit einem Verlängerungsteil S-förmig verlängert sind.

Grundsätzlich wird der für das erfindungsgemäße Wirbelsäulenimplantat verwendete menschliche oder tierische Knochen vor oder nach seiner Bearbeitung in die erfindungsgemäßen Formen einem virusinaktivierenden und sterilisierenden Verfahren unterworfen.

### Bezugzeichenliste

- 10, 10', 10", 10"': Basiskörper
- 12: Vorsprung
- 14: Aussparung
- 20, 20": Verlängerungsteil
- 22: Vorsprung
- 24: Aussparung
- 30: Knochenchips

## Patentansprüche

1. Modulares System zur interkorporellen Fusion an der Wirbelsäule, mit
- zumindest einem Wirbelsäulenimplantat (10, 10', 10", 10"') bestehend aus einem Körper (10, 10', 10", 10"') aus Knochenmaterial, der in Richtung seiner Längserstreckung gekrümmt ausgebildet ist, wobei an einem Ende des Körpers (10, 10'; 10", 10"') ein erstes Kopplungsmittel vorgesehen ist, das einstückig mit dem Körper (10, 10', 10", 10"') ausgebildet ist und das einen ersten Vorsprung (12), welcher von dem einen Ende des Körpers absteht, sowie eine Aussparung (14) aufweist, welche in dem Körper an dem einen Ende ausgebildet ist; und
- mehreren Verlängerungsteilen (20, 20"), die jeweils mit einem zu dem ersten Kopplungsmittel komplementären zweiten Kopplungsmittel (22, 24) versehen sind, das zur Ankopplung an das erste Kopplungsmittel (12, 14) geeignet ist und das einen davon abstehenden zweiten Vorsprung sowie eine zweite darin ausgebildete Ausnehmung aufweist, wobei die Verlängerungsteile unterschiedliche Längen und/oder Krümmungen aufweisen.

2. Modulares System nach Anspruch 1,
**dadurch gekennzeichnet,**
**dass** der Körper (10, 10', 10", 10"') aus kortikalem Knochenmaterial besteht.

3. Modulares System nach einem der vorangehenden Ansprüche,
**dadurch gekennzeichnet,**
**dass** das erste Kopplungsmittel (12, 14) zur formschlüssigen Verbindung mit dem komplementären Kopplungsmittel (22, 24) ausgebildet ist.

4. Modulares System nach einem der vorangehenden Ansprüche,
**dadurch gekennzeichnet,**
**dass** an zumindest einem Ende des Körpers (10, 10', 10", 10"') ein Vorsprung (12) und eine Aussparung (14) vorgesehen sind.

5. Modulares System nach Anspruch 4,
**dadurch gekennzeichnet,**
**dass** an beiden Enden des Körpers jeweils ein Vorsprung (12) und eine Aussparung (14) vorgesehen sind, wobei insbesondere der Vorsprung und die Aussparung an einem Ende in unterschiedlichen Längsebenen des Körpers (10, 10', 10", 10"') angeordnet sind.

6. Wirbelsäulenimplantat nach einem der vorangehenden Ansprüche,
**dadurch gekennzeichnet,**
**dass** bei miteinander verbundenem Körper (10, 10', 10", 10"') und Verlängerungsteil (20, 20") die Krümmung des Körpers stetig in die des Verlängerungsteiles übergeht.

7. Wirbelsäulenimplantat nach Anspruch 6,
**dadurch gekennzeichnet,**
**dass** das Verlängerungsteil (20, 20") ein abgerundetes Ende aufweist.

8. Wirbelsäulenimplantat nach Anspruch 6 oder 7,
**dadurch gekennzeichnet,**
**dass** der Körper (10") und das Verlängerungsteil (20") im Querschnitt keil- oder trapezförmig sind.

9. Wirbelsäulenimplantat nach einem der vorangehenden Ansprüche,
**dadurch gekennzeichnet,**
**dass** der Vorsprung (12) und die Aussparung (14) die gleiche Höhe aufweisen, die vorzugsweise die halbe Höhe des Körpers (10, 10', 10", 10"') beträgt.

10. Wirbelsäulenimplantat nach einem der vorangehenden Ansprüche,
**dadurch gekennzeichnet,**
**dass** der Außenradius des Körpers (10, 10', 10", 10"') mindestens 12 mm und dessen Höhe etwa 6-18 mm beträgt.

## Claims

1. A modular system for interbody fusion at the spinal column comprising
- at least one spinal implant (10, 10', 10", 10"') consisting of a body (10, 10', 10", 10"') made from bone material which is curved in the direction of its longitudinal extent,
wherein a first coupling means is provided at an end of the body (10, 10', 10", 10"'), is made in one piece with the body (10, 10', 10", 10"') and has a first projection (12) protruding from the one end of the body and a cut-out (14) formed at the one end in the body; and
- a plurality of extension parts (20, 20") each provided with a second coupling means (22, 24) which is complementary to the first coupling means, which is suitable for coupling to the first coupling means (12, 14) and which has a second projection protruding therefrom and a second recess formed therein, with the extension parts having different lengths and/or curvatures.

2. A modular system in accordance with claim 1, **characterized in that** the body (10, 10', 10", 10"') consists of cortical bone material.

3. A modular system in accordance with any one of the preceding claims, **characterized in that** the first coupling means (12, 14) is made for shape matched connection to the complementary coupling means (22, 24).

4. A modular system in accordance with any one of the preceding claims, **characterized in that** a projection (12) and a cut-out (14) are provided at at least one end of the body (10, 10', 10", 10"').

5. A modular system in accordance with claim 4, **characterized in that** a respective projection (12) and a respective cut-out (14) are provided at both ends of the body, with in particular the projection and the cut-out being arranged at one end in different longitudinal planes of the body (10, 10', 10", 10"').

6. A spinal implant in accordance with any one of the preceding claims, **characterized in that** the curvature of the body merges constantly into the curvature of the extension part when the body (10, 10', 10", 10"') and the extension part (20, 20") are connected to one another.

7. A spinal implant in accordance with claim 6, **characterized in that** the extension part (20, 20") has a rounded end.

8. A spinal implant in accordance with claim 6 or claim 7, **characterized in that** the body (10") and the extension part (20") are wedge-shaped or trapezoidal in cross-section.

9. A spinal implant in accordance with any one of the preceding claims, **characterized in that** the projection (12) and cut-out (14) have the same height, which preferably amounts to half the height of the body (10, 10', 10", 10"').

10. A spinal implant in accordance with any one of the preceding claims, **characterized in that** the outer radius of the body (10, 10', 10", 10"') amounts to at least 12 mm and its height to approximately 6-18 mm.

## Revendications

1. Système modulaire pour la fusion intercorporelle au niveau de la colonne vertébrale, comportant
- au moins un implant de colonne vertébrale (10, 10', 10", 10"') constitué par un corps (10, 10', 10", 10"') en matériau osseux qui est réalisé incurvé dans la direction de son extension longitudinale, un premier moyen d'accouplement étant prévu à une extrémité du corps (10, 10', 10", 10"'), qui est réalisé d'un seul tenant avec le corps (10, 10', 10", 10"') et qui présente une première saillie (12) dépassant de l'une des extrémités du corps ainsi qu'une échancrure (14) ménagée dans le corps à l'une des extrémités, et
- plusieurs portions de prolongement (20, 20") pourvues chacune d'un second moyen d'accouplement (22, 24) complémentaire au premier moyen d'accouplement, qui convient pour l'accouplement au premier moyen d'accouplement (12, 14) et qui comprend une seconde saillie qui en dépasse ainsi qu'une seconde échancrure ménagée dans celui-ci, les portions de prolongement présentant différentes longueurs et/ou différentes courbures.

2. Système modulaire selon la revendication 1,
**caractérisé en ce que**
le corps (10, 10', 10", 10"') est constitué de matériau osseux cortical.

3. Système modulaire selon l'une des revendications précédentes,
**caractérisé en ce que**
le premier moyen d'accouplement (12, 14) est réalisé pour la liaison en coopération de formes avec le moyen d'accouplement complémentaire (22, 24).

4. Système modulaire selon l'une des revendications précédentes,
**caractérisé en ce que**
une saillie (12) et une échancrure (14) sont prévues au moins à une extrémité du corps (10, 10', 10", 10'").

5. Système modulaire selon la revendication 4,
**caractérisé en ce que**
une saillie respective (12) et une échancrure respective (14) sont prévues aux deux extrémités du corps, en particulier la saillie et l'échancrure à une extrémité étant disposées dans différents plans longitudinaux du corps (10, 10', 10", 10"').

6. Implant de colonne vertébrale selon l'une des revendications précédentes,
**caractérisé en ce que**
lorsque le corps (10, 10', 10", 10"') et la portion de prolongement (20, 20") sont reliés l'un à l'autre, la courbure du corps passe en continu dans celle de la portion de prolongement.

7. Implant de colonne vertébrale selon la revendication 6,
**caractérisé en ce que**
la portion de prolongement (20, 20") présente une extrémité arrondie.

8. Implant de colonne vertébrale selon la revendication 6 ou 7,
**caractérisé en ce que**
le corps (10") et la portion de prolongement (20") ont en section transversale une forme de coin ou de trapèze.

9. Implant de colonne vertébrale selon l'une des revendications précédentes,
**caractérisé en ce que**
la saillie (12) et l'échancrure (14) présentent la même hauteur qui est de préférence la moitié de la hauteur du corps (10, 10', 10", 10"').

10. Implant de colonne vertébrale selon l'une des revendications précédentes,
**caractérisé en ce que**
le rayon extérieur du corps (10, 10', 10"; 10"') est d'au moins 12 mm et sa hauteur est d'environ 6 à 18 mm.
